# EUROPEAN PATENT APPLICATION

(11) **EP 1 721 901 A1**
(43) Date of publication of application: **15.11.2006**
(21) Application number: 05719682.6
(22) Date of filing: 01.03.2005
(51) Int. Cl.: C07D 273/00, A61K 31/395, A61P 31/10, A61P 35/00, A61P 43/00

(54) **ABC TRANSPORTER INHIBITOR**

(30) Priority: 02.03.2004 JP 2004057442
(71) Applicant: SUNTORY LIMITED, Osaka-shi, Osaka 530-8203 (JP)
(72) Inventor: ODA, Kohei, 5941111 (JP); Hiraga, Kazumi, Kyoto Daigaku Shokuin Shukusya, Uji-shi Kyoto 6110011 (JP)
(74) Representative: Vossius & Partner
(86) International application number: PCT/JP2005/003365
(87) International publication number: WO 2005/082870

(57) **Abstract**

It is an object of the present invention to provide an ABC transporter inhibitor whereby the multi-drug resistance against anticancer drugs caused by amplification and expression of MDR1 gene, which is the most serious problem in chemotherapy for cancer, or the resistance against antifungal agents in mycotic diseases can be overcome. The present invention relates to an ABC transporter inhibitor containing enniatin or its analog as the active ingredient. The ABC transporter inhibitor of the present invention, being capable of inhibiting transport/excretion of a drug, especially an anticancer drug or an antifungal agent, from inside to outside of cells, is useful in preventing, suppressing, or inhibiting acquisition of resistance against various drugs.

## Description

### TECHNICAL FIELD

The present invention relates to an ABC transporter inhibitor which contains enniatin or its analog as the active ingredient and inhibits an active transport function of ABC (ATP-binding cassette) transporter. It also relates to an inhibitor of drug-resistance acquisition, which contains enniatin or its analog as an active ingredient.

### BACKGROUND ART

The ABC transporter is a general term for membrane proteins having two ATP-binding regions (NBF) in the molecule and 12 to 18 transmembrane domains driven or regulated by ATP. The ABC transporter is one of the families distributed in a wide range of biological species from bacteria, yeasts, plants, to mammals, and 500 or more ABC transporter-related genes have been identified so far. Particularly in human being, 40 or more ABC transporter genes have been identified up to now, and it has been clarified that abnormality of each gene causes various diseases. Accordingly, the importance of the ABC transporter as a biodefense mechanism has been recognized. Among these ABC transporters, ABC transporters that are involved in the acquisition of multi-drug resistance have recently attracted attention. The ABC transporter involved in the acquisition of multi-drug resistance that was first identified is human and mouse MDR1 (multi-drug resistance; sometimes referred to as P-glycoprotein). This is based on the function named ABC transporter, a mechanism whereby cancer cells acquire resistance to anticancer drugs and a drug is transported from inside to outside of cells utilizing the energy generated by ATP hydrolysis. In this way, the ABC transporter excretes various drugs, in particular anticancer drugs, from inside to outside of cancer cells.

MDR1 was found during the course of study on "acquired multi-drug resistance" which is a problem in cancer therapy where cancer cells acquire resistance simultaneously to many anticancer drugs different in action mechanism and/or chemical structure after administration of an anticancer drug. When the gene amplifying in cultured cells that have acquired strong resistance to an anticancer drug is isolated and allowed to express in drug-sensitive cells, the cells become resistant to various anticancer drugs. Thus the gene was named MDR1 representing the capitals of Multi Drug Resistance (see Non-patent document 1).

It has been clarified that proteins encoded by the MDR1 gene (hereinafter also referred to as MDR1 proteins) function as a pump to excrete various fat soluble drugs from inside to outside of cells utilizing the energy generated by ATP hydrolysis so that the cells become multi-drug resistant.

In addition, MDR1, being expressed in capillary endothelium of brain and testis, placental chorion, membrane at the side of lumen of small intestine and capillary bile duct, renal proximal tubule, hematopoietic stem cells, etc., is believed to function not only to suppress the invasion of harmful foreign substances into body but also to protect important organs such as brain and fetus from abnormal substances coming into blood flow (see Non-patent document 2).

Although MDR1 proteins act as a barrier to harmful substances it cannot excrete all harmful fat-soluble substances. Many of the harmful fat soluble substances that MDR1 proteins cannot excrete are detoxicated by conjugation with glutathione or glucuronic acid in cells and excreted to outside of the cells by a different ABC transporter, the protein (hereinafter also referred to as MRP1 protein) encoded by MRP1 (Multidrug Resistance Protein) gene.

Such MDR1 gene analogs involved in the excretion of foreign substances is not expressed in human being only, but it has been known that, in yeast, for example, many types of ABC transporter genes slightly different from each other in substrate specificity are known to be present (see Non-patent document 3). Probably the function to excrete a foreign substance is important for yeast which is a unicellular organism, and gene duplication may have occurred during the process of evolution.

ABC transporter genes involved in excretion of foreign substances known for budding yeast (yeast belonging to *Saccharomyces*) include PDR5 and SNQ2 closely related to MDR1, STE6 involved in secretion of yeast-mating factor, YCF1 involved in excretion of cadmium, and organic anion transporter gene YRS1/YOR1 (see Non-patent document 4).

Among these genes, the protein encoded by PDR5 gene (hereinafter also referred to as PDR5 protein) is known to excrete various drugs including cycloheximide, cerulenin, compactin, staurosporine, sulfometuron-methyl, trifluoroperazine, rhodamine, etc. to outside of the cells by active transport so as tomake yeast resistant to those drugs (see Non-patent document 5).

Although SNQ2, a homolog of PDR5, is known to be involved in resistance to drugs such as 4-nitoroquinolin N-oxide, only two or three compounds are known as those which can excrete SNQ2 to outside of the cells (see Non-patent document 6).

Recently CDR1 and CDR2 of yeast belonging to *Candida* have been obtained as the genes complementary to the drug sensitivity of the PDRS-deleted strain of yeast belonging to *Saccharomyces.* This suggests that the PDR5 protein may be analogous to the protein encoded by CDR1 or CDR2 gene (hereinafter also referred to as CDR1 or CDR2 protein) of *Candida* yeast in specificity of the substance to transport actively (see Non-patent document 7).

As mentioned above, the ABC transporter is distributed among a wide range of biological species from bacteria to mammals and recognized to be important as a biodefense mechanism. Under these circumstances, a substance that modulates the active transport function of the ABC transporter, i.e. an ABC transporter inhibitor, has been desired and various researches have been done.

ABC transporter inhibitors, especially inhibitors of P-glycoprotein which is the MDR1 protein, have extensively been studied as drugs (multi-drug resistance antagonists) to solve multi-drug resistance caused by expression of P-glycoprotein in cancer cells, which is the most serious problem in chemotherapy for cancer. For example, verapamil which is a calcium antagonist (see Non-patent document 8), cyclosporine A which is an immunosuppressant (see Non-patent document 9), FK-506 which is also an immunosuppressant (see Non-patent document 10), and progesterone which is a steroid(see Non-patent document 11) are being studied as multi-drug resistance antagonists. Each of these compounds, however, is now being used or developed as a drug having an efficacy other than multi-drug resistance antagonism. Therefore, the compound, when used as a multi-drug resistance antagonist, caused a problem that the efficacy resulted in an adverse drug reaction. Then its derivative with reduced original efficacy or a novel ABC transporter inhibitor that can inhibit the function of the P-glycoprotein even at a lower concentration has been investigated, but these are so cytotoxic that development as amulti-drug resistance antagonist was unsuccessful.

Certain cyclic depsipeptides having 18 ring atoms (enniatins) are each known as ionophore antibiotics acting on gram-positive bacteria (see Non-patent document 13). The method for production of these compounds is also known (see, for example, Non-patent document 12).

A cyclic depsipeptide having 18 ring atoms with the same basic skeleton as that of enniatins has been disclosed as a drug for prevention and elimination of endoparasites (see, for example, Patent document 2).

None of the above-mentioned documents, however, describe that enniatins have the ABC transporter inhibiting activity, the activity to inhibit PDR5 protein of *Saccharomyces* yeast, and the activity to inhibit CDR1 and/or CDR2 protein of *Candida* yeast.
Patent document 1: International Publication No.WO2003/8644 pamphlet
Patent document 2: International Publication No.WO93/25543 pamphlet
Non-patent document 1: Ueda, K. and 3 others, Proc. Natl. Acad. Sci. USA, 1987, Vol.84, p.3004-3008
Non-patent document 2: Ueda, K. , Protein, Nucleic Acid and Enzyme, 2001, Vol.46, p.588-595
Non-patent document 3: Miyagawa, T. andTakahashi, H. , Bioscience and Industry, 2000, Vol. 58, P.397-400
Non-patent document 4: Bauer, B.E. et al., Biochim. Biophys. Acta, 1999, Vol.1461, p.217-236
Non-patent document 5: Hirata, D. et al., Curr. Genet., 1994, Vol.26, p.285-294
Non-patent document 6: Decottigniest, A. et al., J. Biol. Chem., 1995, Vol.270, p.18150-18157
Non-patent document 7: Schuetzer, M. et al., J. Antimicrobial Agents, 2003, Vol.22, p.291-300.
Non-patent document 8: Tsuruo, T. et al., Cancer Res., 1981, Vol.41, 1967-1973
Non-patent document 9: Slater, L.M. et al., J. Clin. Invest., 1986, Vol.77, p.1405-1408
Non-patent document 10: Saeki, T. et al. , J. Biol. Chem. , 1993, Vol.268, p.6077-6080
Non-patent document 11: Conseil, G. et al., Biochemistry, 2000, Vol.39, p.6910-6917
Non-patent document 12: Tomoda, H. et al. , J. Antibiotics., 1992, Vol.45, p.1207-1215
Non-patent document 13: Visconti, A. et al. , J. Agric. Food Chem., 1992, Vol.40, p.1076-1082

### DISCLOSURE OF THE INVENTION

The purpose of the present invention is to provide an ABC transporter inhibitor containing a cyclic depsipeptide (hereinafter also referred to as an enniatin) as the active ingredient. More particularly, the purpose is to provide an ABC transporter inhibitor or an inhibitor to inhibit acquisition of drug resistance which contains an enniatin as the active ingredient and by which the multi-drug resistance against anticancer drugs caused by amplification and expression of MDR1 gene, which is the most serious problem in chemotherapy for cancer, or the resistance against antifungal agents in mycotic diseases can be overcome.

"ABC transporter" (also simply referred to as transporter) in this specification is a membrane protein that transports and excretes a drug from inside to outside of cells utilizing the energy generated by ATP hydrolysis. "ABC transporter inhibitor" is a drug that inhibits or suppresses the transport and excretion of drugs etc. from inside to outside of cells by the ABC transporter. "MDR" is the general name of MDR1, MDR2, and MDR3, the genes encoding membrane proteins belonging to ABC transporters, having two ATP-binding sites per molecule, and exerting ATPase activity. "MDR1 protein" means a protein encoded by the gene MDR1, and in more detail, it is a membrane protein expressed as an APC transporter through transcription, translation, and various modifications of MDR1 gene. "MDR protein" , "CDR1 or CDR2 protein" and "PDR5 protein" mean a protein encoded by either one of the MDR family, a protein encoded by CDR1 or CDR2 gene, and a protein encoded by PDR5 gene, respectively, like "MDR1 protein". "Drug resistance" means the reduced sensitivity to a drug or its homolog or analog as a result of repeated intake of the drug, a condition where a higher amount of the drug is required to produce an effect comparable to that obtained previously with a lower amount of the drug: the condition is also simply referred to as "resistance".

The inventors attempted screening of substances that inhibit the active transport function of PDR5 protein, using the drug-sensitive mutated-form of yeast where PDR5 gene originated from yeast had been allowed to express as described in the International Publication No. WO 03/008644 (see Patent document 1). The inventors of the present invention have found by screening of various substances according to the above-mentioned method that certain substances have strong transporter-inhibitory activity and weak cytotoxicity. The inventors also purified the inhibitors and determined the structure of the inhibitors, and finally the inhibitors were confirmed to be enniatin B, enniatin B1 and enniatin D which are known as alkali metal ionophore antibiotics. The inventors have completed the present invention after further researches.

Namely, the present invention relates to:
(1) an ABC transporter inhibitor which comprises as an active ingredient a cyclic depsipeptide, or its optical isomer or racemate of the formula (I): 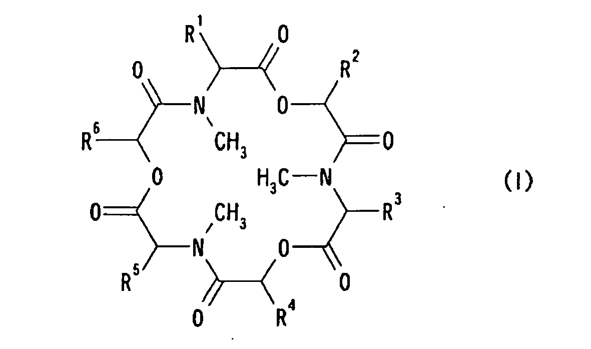 wherein R¹, R³ and R⁵ are each independently a group selected from linear or branched alkyl having up to 8 carbon atoms; hydroxyalkyl; alkanoyloxyalkyl; alkoxyalkyl; aryloxyalkyl; mercaptoalkyl; alkylthioalkyl; alkylsulfinylalkyl; alkylsulfonylalkyl; carboxyalkyl; alkoxycarbonylalkyl; arylalkoxycarbonylalkyl; carbamoylalkyl; aminoalkyl; alkylaminoalkyl; dialkylaminoalkyl; guanidinoalkyl; alkoxycarbonylaminoalkyl; 9-fluorenylmethoxycarbonyl(Fmoc)aminoalkyl; alkenyl; cycloalkyl; cycloalkylalkyl; and arylalkyl optionally substituted with halogen, hydroxy, alkyl, or alkoxy, and R², R⁴ and R⁶ are each independently a group selected from linear or branched alkyl having up to 8 carbon atoms; hydroxyalkyl; alkanoyloxyalkyl; alkoxyalkyl; aryloxyalkyl; alkylthioalkyl; alkylsulfinylalkyl; alkylsulfonylalkyl; carboxyalkyl; alkoxycarbonylalkyl; arylalkoxycarbonylalkyl; carbamoylalkyl; aminoalkyl; alkylaminoalkyl; dialkylaminoalkyl; alkoxycarbonylaminoalkyl; alkenyl; cycloalkyl; cycloalkylalkyl; and aryl or arylalkyl which are optionally substituted with halogen, hydroxy, alkyl, or alkoxy;
(2) the ABC transporter inhibitor according to the above (1), wherein the cyclic depsipeptide is a compound of the formula (II): 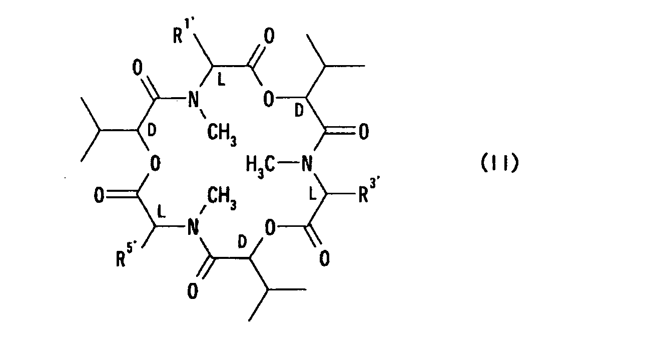 wherein R^{1'}, R^{3'} and R^{5'} are each independently linear or branched lower(C₁₋₄)alkyl;
(3) the ABC transporter inhibitor according to the above (2), wherein the groups represented by R^{1'}, R^{3'} and R^{5'} are linear or branched propyl or butyl;
(4) the ABC transporter inhibitor according to the above (3), wherein R^{1'} and R^{3'} are each isopyropyl, and R^{5'} is any one of the groups selected from isopropyl, sec-butyl, and isobutyl;
(5) the ABC transporter inhibitor according to any one of the above (1) to (4), wherein the ABC transporter is MDR protein;
(6) the ABC transporter inhibitor according to any one of the above (1) to (4), wherein the ABC transporter is CDR1 or CDR2 protein of *Candida* yeast;
(7) the ABC transporter inhibitor according to any one of the above (1) to (4), wherein the ABC transporter is PDR5 protein of *Saccharomyces* yeast;
(8) an inhibitor against the acquisition of drug resistance, which comprises as an active ingredient a cyclic depsipeptide, or its optical isomer or racemate of the formula (I): 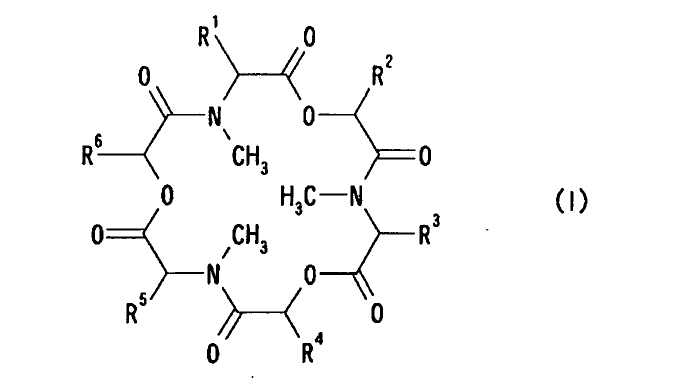
wherein R¹, R³ and R⁵ are each independently a group selected from linear or branched alkyl having up to 8 carbon atoms; hydroxyalkyl; alkanoyloxyalkyl; alkoxyalkyl; aryloxyalkyl; mercaptoalkyl; alkylthioalkyl; alkylsulfinylalkyl; alkylsulfonylalkyl; carboxyalkyl; alkoxycarbonylalkyl; arylalkoxycarbonylalkyl; carbamoylalkyl; aminoalkyl; alkylaminoalkyl; dialkylaminoalkyl; guanidinoalkyl; alkoxycarbonylaminoalkyl; 9-fluorenylmethoxycarbonyl(Fmoc)aminoalkyl; alkenyl; cycloalkyl; cycloalkylalkyl; and arylalkyl optionally substituted with halogen, hydroxy, alkyl, or alkoxy, and R², R⁴ and R⁶ are each independently a group selected from linear or branched alkyl having up to 8 carbon atoms; hydroxyalkyl; alkanoyloxyalkyl; alkoxyalkyl, aryloxyalkyl; alkylthioalkyl; alkylsulfinylalkyl; alkylsulfonylalkyl; carboxyalkyl; alkoxycarbonylalkyl; arylalkoxycarbonylalkyl; carbamoylalkyl; aminoalkyl; alkylaminoalkyl; dialkylaminoalkyl; alkoxycarbonylaminoalkyl; alkenyl; cycloalkyl; cycloalkylalkyl; and aryl or arylalkyl which are optionally substituted with halogen, hydroxy, alkyl, or alkoxy.

The present invention also relates to an ABC transporter inhibitor pharmaceutical composition containing a cyclic depsipeptide, or its optical isomer or racemate, and a pharmaceutically acceptable additive. The present invention also relates to a method for inhibition of ABC transporter by administration of a cyclic depsipeptide, or its optical isomer or racemate to mammals, as well as to use of a cyclic depsipeptide, or its optical isomer or racemate for the production of a drug for inhibition of ABC transporter.

According to another aspect of the present invention, the invention relates to a method to prevent, suppress, or inhibit acquisition of resistance against drugs, especially anticancer drugs and antifungal agents, by administration of an ABC transporter inhibitor containing a cyclic depsipeptide, or its optical isomer or racemate.

The ABC transporter inhibitor of the present invention, being capable of inhibiting transport and excretion of a drug from inside to outside of cells, can prevent, suppress or inhibit acquisition of resistance against various drugs by cells.

The ABC transporter inhibitor of the present invention can inhibit protein transporters of the MDR and MRP (multidrug-resistance-associated protein) families including MDR1, MDR2 , and MDR3. Among these, MDR1 protein excretes various drugs, especially anticancer drugs, from inside to outside of cancer cells. Drugs other than anticancer drugs are exemplified by digoxin, progesterone, and morphine. MDR2 and MDR3 proteins can act as a transporter of phospholipids. In addition the MRP protein family acts as a pump for excretion of various conjugated drugs such as glutathione conjugates or glucuronides. Such a way of transport and/or excretion of a drug from inside to outside of cells is a mechanism by which cells acquire resistance against drugs. Therefore the ABC transporter inhibitor or the drug resistance-acquired inhibitor of the present invention can prevent, suppress or inhibit acquisition of resistance against various drugs.

The ABC transporter inhibitor of the present invention can inhibit the transporter of CDR1 and/or CDR2 protein of *Candida* yeast. CDR1 and/or CDR2 protein of *Candida* yeast transports a drug, particularly an antifungal agent, that was incorporated in *Candida* yeast, to outside the yeast, which is a mechanism by which a fungus acquires resistance against antifungal agents. The ABC transporter inhibitor or a drug resistance-acquired inhibitor of the present invention, being capable of inhibiting the transporter, can prevent, suppress or inhibit acquisition of resistance against antifungal agents in fungi.

The ABC transporter inhibitor of the present invention is also capable of inhibiting the transporter of PDR5 protein of *Saccharomyces* yeast. PRD5 protein of *Saccharomyces* yeast excretes various drugs including cycloheximide, cerulenin, compactin, staurosporine, sulfometuron-methyl, trifluoroperazine, and rhodamine to outside of cells via active transport, and thus yeast acquires resistance against these drugs. Because PDR5 protein of *Saccharomyces* yeast is known to have substrate specificity similar to that of CDR1 and/or CDR2 protein of Candida yeast, the ABC transporter inhibitor or the drug resistance-acquired inhibitor of the present invention can also be used as an inhibitor of CDR1 and/or CDR2 protein of *Candida* yeast, namely as a drug to overcome drug resistance of yeast such as *Candida albicans.*

Further, acquisition of resistance against the above-mentioned drugs may decrease or cancel the efficacy of each drug. The ABC transporter inhibitor or the drug resistance-acquired inhibitor of the present invention can make the drug restore its efficacy that was decreased or cancelled.

Furthermore, the ABC transporter inhibitor of the present invention inhibits release of various cytotoxic substances generated during alcohol fermentation by *Saccharomyces* yeast to suppress the growth of yeast cells. As a result of this, the metabolic system in yeast cells is shifted to alcohol fermentation rather than to cell growth so that alcohol production is increased. Namely, the ABC transporter inhibitor of the present invention can be used as an accelerator of alcohol fermentation by *Saccharomyces* yeast.

### BRIEF DESCRIPTION OF THE DRAWINGS

Fig. 1 illustrates the method for determination of the ABC transporter-inhibitory activity.
Fig. 2 illustrates the calibration curve prepared by plotting the diameter of the inhibitory circle against the logarithm of cycloheximide concentration.

### BEST MODE FOR CARRYING OUT THE INVENTION

In the preferred compounds of the above-mentioned formula (I), the preferred examples of R¹, R³, and R⁵ in the formula are each independently selected from linear or branched C₁₋₈-alkyl, particularly methyl, ethyl, propyl, isopropyl, butyl, isobutyl, sec-butyl, tert-butyl, pentyl, isopentyl, sec-pentyl, hexyl, isohexyl, sec-hexyl, heptyl, isoheptyl, sec-heptyl, tert-heptyl, octyl, isooctyl, and sec-octyl; hydroxy-C₁₋₆-alkyl, particularly hydroxymethyl and 1-hydroxyethyl; C₂₋₄-alkanoyloxy-C₁₋₆-alkyl, particularly, acetoxymethyl and 1-acetoxyethyl; C₁₋₄-alkoxy-C₁₋₆-alkyl, particularly methoxymethyl and 1-methoxyethyl; aryloxy-C₁₋₆-alkyl, particularly phenoxymethyl; mercapto-C₁₋₆-alkyl, particularly mercaptomethyl; C₁₋₄-alkylthio-C₁₋₆-alkyl, particularly methylthioethyl; C₁₋₄-alkylsulfinyl-C₁₋₆-alkyl, particularly methylsulfinylethyl; C₁₋₄-alkylsulfonyl-C₁₋₆-alkyl, particularly methylsulfonylethyl; carboxy-C₁₋₆-alkyl, particularly carboxymethyl and carboxyethyl; C₁₋₄-alkoxycarbonyl-C₁₋₆-alkyl, particularly methoxycarbonylmethyl and ethoxycarbonylethyl; aryl-C₁₋₄-alkoxycarbonyl-C₁₋₆-alkyl, particularly benzyloxycarbonylmethyl; carbamoyl-C₁₋₆-alkyl, particularly carbamoylmethyl and carbamoylethyl; amino-C₁₋₆-alkyl, particularly aminopropyl and aminobutyl; C₁₋₄-alkylamino-C₁₋₆-alkyl, particularly methylaminopropyl and methylaminobutyl; di-C₁₋₄-alkylamino-C₁₋₆-alkyl, particularly dimethylaminopropyl and dimethylaminobutyl; guanidino-C₁₋₆-alkyl, particularly guanidinopropyl; C₁₋₄-alkoxycarbonylamino-C₁₋₆-alkyl, particularly tert-butoxycarbonylaminopropyl and tert-butoxycarbonylaminobutyl; 9-fluorenylmethoxycarbonyl(Fmoc)amino-C₁₋₆-alkyl, particularly 9-fluorenylmethoxycarbonyl(Fmoc)aminopropyl and 9-fluorenylmethoxycarbonyl(Fmoc)aminobutyl; C₂₋₈-alkenyl, particularly vinyl, allyl, and butenyl; C₃₋₇-cycloalkyl, particularly cyclopentyl, cyclohexyl, and cycloheptyl; C₃₋₇-cycloalkyl-C₁₋₄-alkyl, particularly cyclopentylmethyl, cyclohexylmethyl, and cycloheptylmethyl; and aryl-C₁₋₄-alkyl, particularly phenylmethyl (optionally substituted with radical(s) selected from the group consisting of halogen, particularly fluorine, chlorine, bromine, or iodine; hydroxy; alkyl, particularly C₁₋₄-alkyl; and alkoxy, particularly C₁₋₄-alkoxy), among which C₁₋₄-alkyl is particularly preferred, and isopropyl, isobutyl, and sec-butyl is more particularly preferred.

The preferred examples of R², R⁴, and R⁶ are each independently selected from linear or branched C₁₋₈-alkyl, particularly methyl, ethyl, propyl, isopropyl, butyl, isobutyl, tert-butyl, tert-butyl, pentyl, isopentyl, tert-pentyl, hexyl, isohexyl, tert-hexyl, heptyl, isoheptyl, tert-heptyl, tert-heptyl, octyl, isooctyl, and tert-octyl; hydroxy-C₁₋₆-alkyl, particularly hydroxymethyl and 1-hydroxyethyl; C₂₋₄-alkanoyloxy-C₁₋₆-alkyl, particularly acetoxymethyl and 1-acetoxyethyl; C₁₋₄-alkoxy-C₁₋₆-alkyl, particularly methoxymethyl and 1-methoxyethyl; aryloxy-C₁₋₆-alkyl, particularly phenyloxymethyl; C₁₋₄-alkylthio-C₁₋₆-alkyl, particularly methylthioethyl; C₁₋₄-alkylsulfinyl-C₁₋₆-alkyl, particularly methylsulfinylethyl; C₁₋₄-alkylsulfonyl-C₁₋₆-alkyl, particularly methylsulfonylethyl; carboxy-C₁₋₆-alkyl, particularly carboxymethyl and carboxyethyl; C₁₋₄-alkoxycarbonyl-C₁₋₆-alkyl, particularly methoxycarbonylmethyl and ethoxycarbonylethyl; aryl-C₁₋₄-alkoxycarbonyl-C₁₋₆-alkyl, particularly benzyloxycarbonylmethyl; carbamoyl-C₁₋₆-alkyl, particularly carbamoylmethyl and carbamoylethyl; amino-C₁₋₆-alkyl, particularly aminopropyl and aminobutyl; C₁₋₄-alkylamino-C₁₋₆-alkyl, particularly methylaminopropyl and methylaminobutyl; di-C₁₋₄-alkylamino-C₁₋₆-alkyl, particularly dimethylaminopropyl and dimethylaminobutyl; C₁₋₄-alkoxycarbonylamino-C₁₋₆-alkyl, particularly methoxycarbonylaminomethyl and diethoxycarbonylaminomethyl; C₂₋₈-alkenyl, particularly vinyl, allyl, and butenyl; C₃₋₇-cycloalkyl, particularly cyclopentyl, cyclohexyl, and cycloheptyl; C₃₋₇-cycloalkyl-C₁₋₄-alkyl, particularly cyclopentylmethyl, cyclohexylmethyl, and cycloheptylmethyl; aryl, particularly phenyl; and aryl-C₁₋₄-alkyl, particularly phenylmethyl (said phenyl or phenylmethyl can optionally be substituted with radical (s) selected from the group consisting of halogen, particularly fluorine, chlorine, bromine, or iodine; hydroxy; alkyl, particularly C₁₋₄-alkyl; and alkoxy, particularly C₁₋₄-alkoxy), among which C₁₋₄-alkyl is particularly preferred, and isopropyl is more particularly preferred. In the present invention, the compound (I) can exist in the form of optically active stereoisomer or racemate, and all of these can be used.

Particularly preferred compounds of the formula (I) are those wherein R¹, R³, and R⁵ are each independently linear or branched lower(C₁₋₄)-alkyl, particularly methyl, ethyl, propyl, isopropyl, butyl, isobutyl, sec-butyl, and tert-butyl, and R², R⁴, and R⁶ are isopropyl, and their optical isomers and racemates. More preferred compounds of the formula (I) are those of the formula (II). Among the compounds of the formula (II), enniatins A, A1, B, B1, C, D, E, and F are preferred, and enniatins A, A1, B, B1, and D are particularly preferred among enniatins shown in Table 1.

**Table 1**

| | | | |
|---|---|---|---|
| 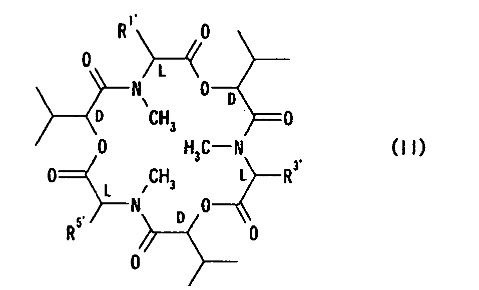 | | | |

| Compound | R^{1'} | R^{3'} | R^{5'} |
|---|---|---|---|
| Enniatin A | sec-butyl | sec-butyl | sec-butyl |
| Enniatin A1 | Isopropyl | sec-butyl | sec-butyl. |
| Enniatin B | Isopropyl | Isopropyl | Isopropyl |
| Enniatin B1 | Isopropyl | Isopropyl | sec-butyl |
| Enniatin C | Isobutyl | Isobutyl | Isobutyl |
| Enniatin D | Isopropyl | Isopropyl | Isobutyl |
| Enniatin E | Isopropyl | Isobutyl | sec-butyl |
| | Isopropyl | sec-butyl | Isobutyl |
| Enniatin F | Isobutyl | sec-butyl | sec-butyl |

Enniatins as the active ingredient of the ABC transporter inhibitor of the present invention can be obtained by purification according to the publicly known method (see, for example, Non-patent document 12, Madry, N. , et al., Eur. J. Appl. Microbiol. Biotechnol., 1983, Vol.17, p.75-79, Matthias Herrmann, et al., Appl. Environ. Microbiol., 1996, p.393-398, etc.). They can be synthesized according to or in a similar manner to the method described in Patent document 2.

The mixture of enniatins A, A1, B and B1 is available from SIGMA-ALDRICH.

The ABC transporter inhibitor of the present invention can inhibit the drug transport of MDR proteins such as MDR1 protein, MDR2 protein, and MDR3 protein, particularly MDR1 protein, and of an MRP (multidrug-resistance-associated protein). The MDR1 protein is expressed in cancer cells and excretes many anticancer drugs to outside of cells to make cancer cells resistant against drugs. The MDR1 protein is expressed not only in cancer cells but also in normal organs (liver, small intestine, cerebrovascular endothelial cells, etc.) and involved in transport of substances. Drugs other than anticancer drugs include steroids such as digoxin, progesterone, cortisol, and aldosterone, as well as morphine. MDR2 protein and MDR3 protein can act as a transporter of phospholipids. The MRP protein family acts as a pump to excrete various glutathione-conjugated drugs or glucuronides of drugs.

These transporters constitute a primary mechanism by which various cells acquire resistance against various drugs, for example, cancer cells acquire resistance against anticancer drugs. Therefore, the ABC transporter inhibitor of the present invention can prevent, suppress or inhibit acquisition of resistance against various drugs by cells.

The above-mentioned anticancer drugs are exemplified by, though not limited to, alkylating agents, antimetabolites, antitumor antibiotics, other antitumor agents, antitumor plant components, BRM (biological response modifiers), angiogenesis inhibitors, cell adhesion inhibitors, and matrix-metalloprotease inhibitors, etc. The alkylating agents include, for example, a chloroethylamine series of alkylating agents such as nitrogen mustard, nitrogen mustard N-oxide, ifosfamide, melphalan, cyclophosphamide, and chlorambucil; an aziridine series of alkylating agents such as carboquone and thiotepa, etc.; an epoxide series of alkylating agents such as dibromomannitol and dibromodulcitol, etc.; a nitrosourea series of alkylating agents such as carmustine, lomustine, semustine, nimustinehydrochloride, chlorozotocin, andranimustine, etc.; sulfonic acid esters such as busulfan, improsulfan tosilate, and piposulfan; dacarbazine and procarbazine, etc.. The antimetabolites include, for example, purine antimetabolites such as 6-mercaptopurine, azathiopurine, 6-thioguanine, and thioinocine, etc.; pyrimidine antimetabolites such as fluorouracil, tegafur, tegafururacil, carmofur, doxifluridine, brocusuridine, cytarabine, and enocitabine, etc.; folic acid antimetabolites such as methotrexate and trimethotrexate,etc.; and salts or complexes thereof. The antitumor antibiotics are exemplified by those of anthracycline series such as daunorubicin, aclarubicin, doxorubicin, pirarubicin, and epirubicin, etc.; those of actinomycin series such as actinomycin D; those of chromomycin series such as chromomycin A₃; those of mitomycin series such as mitomycin C, etc.; those of bleomycin series such as bleomycin and peplomycin, etc.; and salts or complexes thereof. Other antitumor agents include, for example, cysplatin, carboplatin, tamoxifen, L-asparaginase, aceglatone, sizofiran, picibanil, ubenimex, and krestin, etc., and salts or complexes thereof. The antitumor plant components include, for example, plant alkaloids such as camptotecin, vindesine, vincristine, and vinblastine, etc.; epipodophyllotoxines such as etoposide and teniposide, etc.; and salts or complexes thereof. Additional examples are pipobroman, neocarzinostatin, hydroxyurea, etc. BRMs are exemplified by tumor necrosis factor, indometacin, etc. and salts or complexes thereof.

The ABC transporter inhibitor of the present invention can also inhibit transport of substances by proteins expressed by CDR1 and/or CDR2 gene of the above-mentioned *Candida* yeast. *Candida albicans* is known as the yeast that causes mycosis including candidiasis. CDR1 and/or CDR2 proteins of *Candida* yeast transport and excrete a drug incorporated in *Candida* yeast, particularly an antifungal agent, to outside of the yeast. This is the primary mechanism by which *Candida* yeast acquires resistance against antifungal agents. Therefore the ABC transporter inhibitor of the present invention can prevent, suppress or inhibit acquisition of resistance to various antifungal agents by *Candida* yeast and various fungi.

The antifungal agents are exemplified by amphotericin B, butoconazole nitrate, ketoconazole, econazole, etretinate, fluconazole, flucytosine, griseofulvin, itraconazole, miconazole nitrate, nystatin, sulconazole, tioconazole, metronidazole, tinidazole, bifonazole, clotrimazole, and terbinafine hydrochloride.

PDR5 protein of *Saccharomyces* yeast is known to excrete various drugs including cycloheximide, cerulenin, compactin, staurosporine, sulfometuron-methyl, trifluoroperazine and rhodamine to outside of cells via the active transport so as to make yeast resistant to these drugs. Therefore, the ABC transporter inhibitor of the present invention can prevent, suppress or inhibit acquisition of resistance against the above-mentioned drugs including cycloheximide. Because PDR5 protein of *Saccharomyces* yeast is known to be similar to CDR1 and/or CDR2 protein of *Candida* yeast in substrate specificity, the ABC transporter inhibitor of the present invention can inhibit transport of substances by CDR1 and/or CDR2 protein of *Candida* yeast.

The ABC transporter inhibitor of the present invention inhibits extracellular release by *Saccharomyces* yeast of various cytotoxic substances produced during alcohol fermentation to suppress cell growth of yeast. As a result of this, the metabolic system in yeast cells may be shifted from cell growth rather to alcohol fermentation so that alcohol production can be increased. If fermentation rate could be increased, cost of production would be reduced. Because also metabolism other than glycolysis in yeast can greatly be changed as a result of change of the type and assimilation rate of sugar, components of flavor which is very important for liquors can be affected variously. The ABC transporter inhibitor of the present invention can be used for modification of the odor of liquors as a result of inhibition of the transporter.

The ABC transporter inhibitor of the present invention is preferably used together with the above-mentioned drugs against which resistance may be produced. For example, in diseases to be treated with an anticancer drug or antifungal agent, concomitant use of the ABC transporter inhibitor with the anticancer drug or antifungal agent is preferable. Diseases to be treated with an anticancer drug includes, for example, breast cancer, lung cancer, colon cancer, liver cancer, kidney cancer, pancreas cancer, prostate cancer, ovary cancer, cervix cancer, uterine cancer, urinary bladder cancer, brain tumor, adrenal cancer, multiple myeloma, otolaryngeal cancer (including cancer of esophagus, larynx, and pharynx), leukemia, lymphoma, sarcoma, and carcinoid tumor. Diseases to be treated with an antifungal agent include skin mycosis (trichophytosis (trichophytia pompholyciformis, trichophytia eczematosa marginata, trichophytia capitis, etc.)), subcutaneous mycosis (mycetoma, actinomycosis, sporotrichosis, chromomycosis, etc.), mucosal mycosis (candidiasis, aspergillosis, mucormycosis, etc.), lung mycosis (cryptococcosis, histoplasmosis, blastomycosis, coccidioidomycosis, paracoccidioidomycosis, etc.), lung coccidioidomycosis (coccidiodomycosis), lung histoplasmosis (histoplasmosis), blastomycosis, and paracoccidioidomycosis.

The ABC transporter inhibitor of the present invention can be used in mammals (for example, human, mouse, rat, rabbit, dog, cat, cow, horse, pig, monkey, etc.).

The dose of the ABC transporter inhibitor of the present invention is dependent on various factors including the type of disease, seriousness of the condition, age, body weight, and sex of the patient, type of the drug used, and absence/presence of acquisition of resistance. An experienced clinician will be able to determine or prescribe easily the effective dose of a compound or a pharmaceutical composition necessary for diagnosis or treatment of the patient. For convenience, it is desirable for those skilled in the art to begin with a relatively low dose followed by escalation of the dose till the maximum response is achieved.

The ABC transporter inhibitor of the present invention is produced in the form of an enniatin as it is, or in the form of a pharmaceutical composition with a publicly known pharmaceutically acceptable carrier, etc.

The above-mentioned pharmaceutical composition may be in a publicly known drug form, for example, a solid preparation such as tablets, capsules, granules, and powders, and a liquid preparation such as syrup and injection. The ABC transporter inhibitor of the present invention can be administered orally or parenterally.

The above-mentioned pharmaceutically acceptable carriers are various organic or inorganic carriers which are commonly used as materials for preparations and compounded as an excipient, lubricant, binder, and disintegrator for solid preparations; and as a solvent, solubilizer, suspending agent, isotonic agent, buffer, soothing agent, etc. for liquid preparations. A formulation additive such as an antiseptic, stabilizer, colorant, and sweetener may be used as needed.

Preferred examples of excipients include lactose, sucrose, D-mannitol, starch, crystalline cellulose, and light anhydrous silicic acid, and the like. Preferred examples of lubricants include magnesium stearate, calcium stearate, talc, colloidal silica, and magnesium silicatem, and the like. Preferable examples of binders include crystalline cellulose, sucrose, D-mannitol, dextrin, hydroxypropyl cellulose, hydroxypropylmethyl cellulose, polyvinylpyrrolidone, acacia, carmellose, and simple syrup, and the like. Preferred examples of disintegrators include carmellose, carmellose calcium, starch, and crystalline cellulose, and the like. Preferred examples of solvents include water for injection, purifiedwater, alcohol, propylene glycol, macrogol, sesame oil, and corn oil, and the like. Preferred examples of solubilizers include polyethyleneglycol, propylene glycol, benzyl benzoate, cholesterol, sodium benzoate, and citric acid, and the like. Preferred examples of suspending agents include polyvinyl alcohol, polyvinylpyrrolidone, sodium carboxymethylcellulose, methylcellulose, hydroxypropylmethyl cellulose, hydroxyethyl cellulose, hydroxypropyl cellulose, acacia, carmellose, and gelatin, and the like. Preferred examples of isotonic agents include sodium chloride, glycerin, glucose, and D-mannitol, and the like. Preferred examples of buffers include citric acid, sodium citrate, sodium hydrogen carbonate, acetic acid, sodium acetate, sodium hydrogen phosphate, lactic acid, boric acid and borax, and the like. Preferred examples of soothing agents include benzyl alcohol, and the like. Preferred examples of antiseptics include p-hydroxybenzoic acid esters, chlorobutanol, benzyl alcohol, phenethyl alcohol, dehydroacetic acid, sorbic acid, benzalkonium chloride, and benzethonium chloride, and the like. Preferred examples of stabilizers include sodium hydrogen sulfite, sodium pyrosulfite, ascorbic acid, tocopherol, and disodium edentate, and the like. Preferred examples of colorants include titanium oxide, and the like. Preferred examples of sweeteners include fructose, D-sorbitol, glucose, simple syrup, aspartame, and potassium acesulfam, and the like.

In the pharmaceutical composition of the present invention, a drug that causes resistance such as the above-mentioned anticancer drugs can be compounded with an enniatin. By compounding, acquisition of resistance against the drug compounded can easily be prevented, suppressed or inhibited.

In the pharmaceutical composition of the present invention, an additional active ingredient of the same or different class may be contained as needed as far as the addition is appropriate for the purpose of the present invention.

The activity of the ABC transporter inhibitor of the present invention can be determined, for example, by the method described in the International Publication No. WO 03/008644 (see Patent document 1).

The present invention will be further described by means of the following Examples, but not limited to the Examples.

Unless otherwise indicated, "%" as used in the present invention refers to "% (W/V)".

### Example 1

### ABC transporter inhibitory activity

### (1) Drugs tested

An enniatin mixture (mixture of A, A1, B, and B1: SIGMA-ALDRICH), and enniatins B, B1, and D were used. The following enniatins B, B1, and D were used.

### Enniatin B:

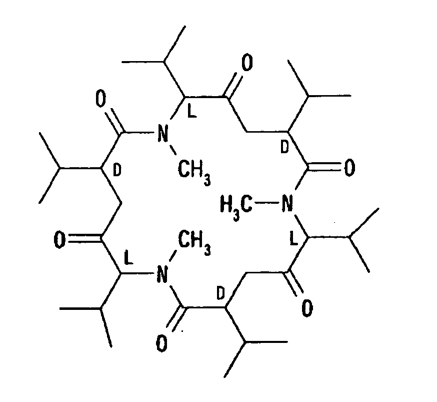

Molecular weight: 639, Chemical formula: C₃₃H₅₇N₃O₉
¹³C-NMR (150MHz), ¹H-NMR (600-MHz):

**Table 2**

| Position | ¹³C-NMR δC | ¹H NMR δH |
|---|---|---|
| Val α-CH | 63.3 | 4.52 |
| Val β-CH | 27.9 | 2.29 |
| Val γ-CH₃ | 19.2, 20.5 | 1.06, 0.89 |
| Hiv α-CH | 75.7 | 5.13 |
| Hiv β-CH | 29.9 | 2.29 |
| Hiv γ-CH₃ | 18.5, 18.7 | 0.98 |
| N-CH₃ | 33.4 | 3.13 |
| CO-N | 169.1 | |
| CO-O | 170.2 | |

Enniatin B1: 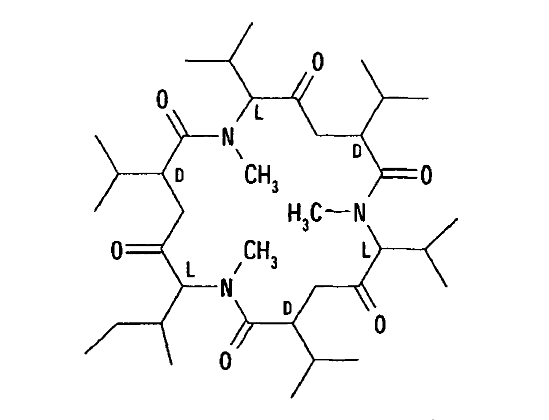 Molecular weight: 653, Chemical formula: C₃₄H₅₉N₃O₉
¹H-NMR (600MHz):

**Table 3**

| Position | ¹H NMR δH |
|---|---|
| Val α-CH | 4.49 |
| Val β-CH | 2.28 |
| Val γ-CH₃ | 1.08, 0.84 |
| Ile α-CH | 4.73 |
| Ile β-CH | ND |
| Ile γ-CH₂ | 1.42, 1.06 |
| Ile γ-CH₃ | 0.95 |
| Ile δ-CH₃ | 0.83 |
| Hiv α-CH | 5.11 |
| Hiv β-CH | 2.28 |
| Hiv γ-CH₃ | 0.89, 0.99 |
| N-CH₃ | 3.11, 3.20 |

Enniatin D: 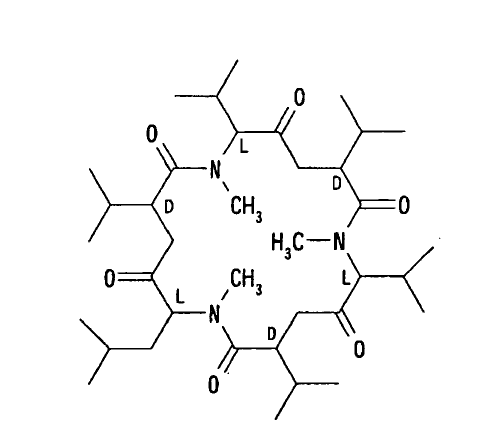 Molecular weight: 653, Chemical formula: C₃₄H₅₉N₃O₉
¹H-NMR (600MHz):

**Table 4**

| Position | ¹H NMR δH |
|---|---|
| Val α-CH | 4.95 |
| Val β-CH | 4.46 |
| Val γ-CH₃ | 1.04, 0.87 |
| Leu α-CH | 4.68 |
| Leu β-CH₂ | 1.74, 1.83 |
| Leu γ-CH | 1.51 |
| Leu δ-CH₃ | ND |
| Hiv α-CH | 5.11 |
| Hiv β-CH | 2.25 |
| Hiv γ-CH₃ | 0.92, 0.98 |
| N-CH₃ | 3.08, 3.13, 3.17 |

### (2) Test method

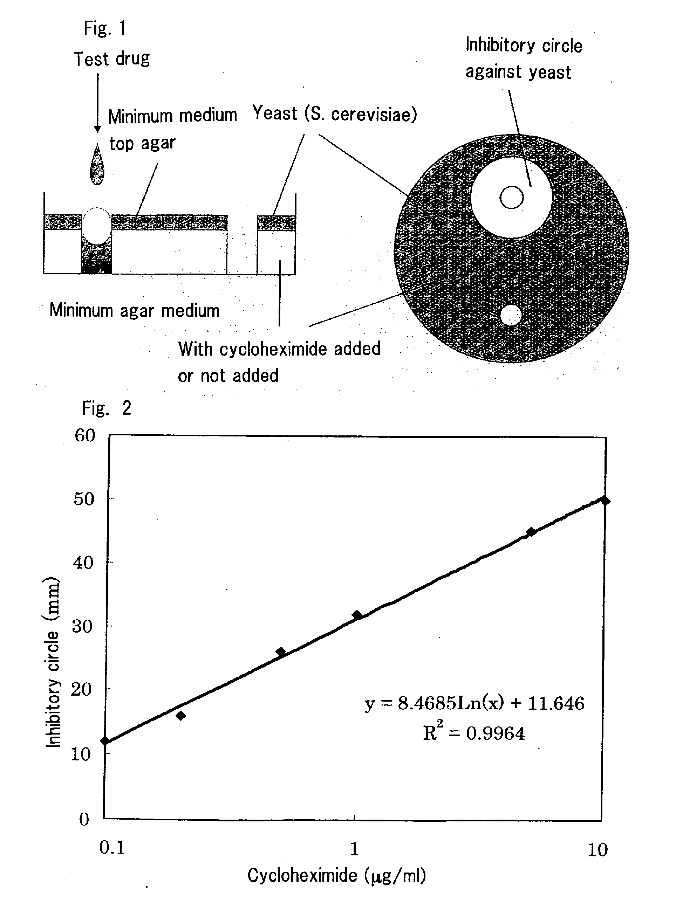

As shown in Fig. 1, a minimum agar medium plate (1.5% agar) containing cycloheximide to the concentration of 0.04% was prepared, and holes of 8 mm in diameter were made with a cork borer. The agar in each hole was removed with tweezers, and a solution of the test drug was poured into the hole. Yeast with overexpressed PDR5 was inoculated to 4 mL of the minimum liquid medium. After shaking culture at 30°C for 12 hours, 4×10⁶ cells were inoculated to 4 mL of the minimum medium top agar, which was thoroughly mixed and added to the minimum agar medium, thereby to give the upper layer. Then static culture at 30°C for 2 days was performed. During this culture, an inhibitory circle against yeast (Halo) will be formed when the test drug is a substance which inhibits the yeast PDR5 protein or a substance which is inhibitory to the growth of yeast. Yeast with overexpressed PDR5 was obtained according to the method described in the International Publication No. WO 03/008644. Namely, the yeast was prepared using *Saccharomyces cerevisiae* W303 (MAT a Δsyr/erg3::HIS3 Δpdr5::LEU2 Δsnq2::HIS3), which had been made drug-sensitive by deletion of SYR/ERG3 gene involved in the synthesis of membrane ergosterol and of which PDR5 and SNQ2 had also been deleted, as the host, and by transformation with an expression vector that makes PDR5 express efficiently. The method for preparation of the expression vector and the method for transformation were also in accordance with the methods described in the International Publication No. WO 03/008644.

Cycloheximide, as a reference substance, at various concentrations was poured into the holes, and the diameter of the inhibitory circle formed was plotted against the concentration of cycloheximide to prepare the calibration curve (Fig. 2). Under these conditions, the inhibitory unit (IU) for formation of an inhibitory circle of 20 mm in diameter was defined as 20 IU/mL per mL, and the inhibitory concentration of the test drug was calculated using the calibration curve.

The following minimum medium was used. The minimum agar medium was prepared by adding agar to 1.5% to the minimum medium and the minimum medium top agar was prepared by adding agar to 1% to the minimum medium.
Minimum medium:

The minimum medium of the following composition was prepared.

**Table 5**

| | |
|---|---|
| Potassium dihydrogen phosphate | 0.15% |
| Calcium chloride dihydrate | 0.01% |
| Magnesium sulfate heptahydrate | 0.05% |
| Ammonium sulfate | 0.2% |
| Trace element stock | 0.01% |
| Glucose | 2% |
| 0.5% Potassium iodide solution | 0.02% |
| Adenine (2 mg/mL) | 1% |
| Leucine (3 mg/mL) | 1% |
| Histidine (2 mg/mL) | 1% |
| Tryptophan (2 mg/mL) | 5% |
| Vitamin mixture | 5% |

### Example 2

### Inhibition of PDR5 protein

Yeast with overexpressed PDR5 by transformation of the host yeast *(Saccharomyces cerevisiae* W303 (MAT a Δsyr/erg3::HIS3 Δpdr5::LEU2 Δsnq2::HIS3)) with the PDR5 expression vector and the host yeast were inoculated separately in 4 mL each of the minimum medium followed by shaking culture at 30°C for 12 hours. To a 96-well microtiter plate to which cycloheximide or cerulenin of various concentrations had been added, was added purified enniatinB, B1, D, or enniatin mixture of various concentrations, followed by inoculation of the above-mentioned yeast. The volume per well was adjusted to 200 µL by addition of the minimum medium. After static culture at 30°C for 24 hours, the extent of growth of yeast was determined for evaluation of the growth-inhibiting activity of the test drug against the yeast with overexpressed PDR5 and against the host yeast. The extent of growth of yeast was determined by measurement of absorbance at 660 nm using a microtiter plate reader (MTP-500; CORONA). The growth-inhibiting activity against the yeast with overexpressed PDR5 and against the host yeast was also determined for immunosuppressor FK-506, the positive control, which is known to inhibit yeast PDR5 protein and human MDR1 protein (P-glycoprotein).

Each of enniatins B, B1, D, and enniatin mixture inhibited the growth of the yeast with overexpressed PDR5 in a concentration-dependent manner, and inhibited growth of the yeast with overexpressed PDR5 completely at the concentration of 5 µg/mL. Inhibition of growth of the host yeast was not observed even when the concentration of the above-mentioned test drug was elevated to 5 µg/mL. This result confirmed that enniatins B, B1, D, and enniatin mixture are the desired ABC transporter inhibitors having no cytotoxicity because they inhibited completely PDR5 protein at the concentration of 5 µg/mL and did not inhibit growth of the host yeast at this concentration.

As for PDR5 protein-inhibiting activity of enniatin mixture as compared with that of FK-506, the ratio (increment of sensitivity) between the concentration for 50% growth-inhibition (IC₅₀) in the presence of cycloheximide with addition of enniatin mixture at 5 µg/mL and the IC₅₀ without addition was higher than that with FK506. The increment of sensitivity to cerulenin was also higher with enniatin mixture than with FK-506 (Table 6). Based on these results, it could be confirmed that enniatin mixture has a stronger activity to inhibit PDR5 protein than FK-506.

**Table 6**

| Drug | IC₅₀ (µg/mL) | Increment of sensitivity |
|---|---|---|
| Cycloheximide | 0.130 | |
| Cycloheximide+5µg/mL enniatin mixture | 0.017 | 7.65 |
| Cerulenin | 0.351 | |
| Cerulenin + 5 µg/mL enniatin mixture | 0.102 | 3.44 |
| Cycloheximide | 0.122 | |
| Cycloheximide + 5.0 µg/mL FK-506 | 0.031 | 3.94 |
| Cerulenin | 0.336 | |
| Cerulenin + 5.0 µg/mL FK-506 | 0.169 | 1.99 |

### Example 3

### Mechanism for PDR5 protein inhibition by enniatin

Rhodamine 6G is a fluorescent substance which has been confirmed to be excreted by PDR5 protein, and in the above-mentioned host yeast with PDR5 deleted, rhodamine 6G will be accumulated in the yeast cells so that the cells exhibit strong fluorescence whereas only weak fluorescence is noted in the above-mentioned yeast with overexpressed PDR5 where rhodamine 6G is excreted outside the cells. If enniatin should inhibit the function of PDR5 protein, rhodamine 6G would be also accumulated in the cells of the yeast with overexpressed PDR5 so that the yeast cells would exhibit fluorescence. The above-mentioned strain with overexpressed PDR5 and the above-mentioned host yeast were separately subjected to shaking culture at 30°C for 12 hours in 4 mL of the minimum medium. Then a quantity of yeast corresponding to 1.0×10⁶ cells was inoculated to 4 mL of the minimum medium . Enniatin B, B1, D, enniatin mixture, or FK-506 was added to the final concentration of 25 µg/mL, and after shaking culture at 30°C for 2 hours, 15 µL of rhodamine 6G (1 mg/mL) was added followed by culture under the same conditions for 30 minutes. After culture, the culture was washed twice with 2% glucose solution in PBS (phosphate-buffered saline) and the fluorescence in yeast cells was observed under a fluorescence microscope (Nicon ECLIPSE E400, using G-2A filter).

Yeast with overexpressed PDR5 where enniatin B, B1, D, enniatin mixture, or FK-506 had been added to the strain with overexpressed PDR5 similarly accumulated rhodamine 6G in any case. When enniatin B, B1, D, enniatin mixture, or FK-506 was added to the PDRS-deleted host yeast, rhodamine 6G was accumulated in yeast cells so that fluorescence was exhibited as in the case of no addition of the drug. These results confirmed that enniatins B, B1, D, enniatin mixture, and FK-506 inhibit the function of PDR5 protein.

The effect of enniatin on expression of PDR5 gene was analyzed based on measurement of the PDR5 transcription activity using β-galactosidase gene as the reporter gene. Yeast, where had been introduced a recombinant plasmid obtained by fusion of β-galactosidase gene (lacZ) in the downstream of Pdr5 promoter, was subjected to shaking culture at 30°C for 8 hours in 4 mL of YEPD medium, and a quantity of yeast corresponding to 1.0×10⁶ cells was inoculated in 4 mL of the medium. At the same time, enniatin B, B1, D, enniatin mixture, or FK-506 was added to the final concentration of 25 µg/mL. After shaking culture at 30°C for 8 hours, 1.0 × 10⁷ yeast cells were collected. The cells were then suspended in 1 mL of Z buffer solution (60 mM disodium hydrogen phosphate, 40 mM sodium dihydrogen phosphate, 10 mM potassium chloride, 1 mM magnesium sulfate, 50 mM methylmercaptan, pH 7.0). The cell suspension was treated with 45µL of chloroform and 30 µL of 0.1% sodium dodecyl sulfate at 30°C for 5 minutes. The suspension was then treated with 200 µL of o-NPG (o-nitrophenyl-β-D-galactopyranoside) of 4 mg/mL at 30°C for 10 minutes. After reaction was stopped by addition of 500 µL of 1 M sodium carbonate, absorbance at 420 nm was measured to determine the activity of β-galactosidase. The activity of β-galactosidase was calculated using a unit = 1000 × A420/10 (reaction time). The activity of β-galactosidase after addition of enniatin B, B1, D, enniatin mixture, or FK-506 was not different from that without addition of the drug. Thus it could be confirmed that inhibition of PDR5 function by enniatin B, B1, D, enniatin mixture, or FK-506 is not ascribable to the inhibition of transcription of PDR5 gene.

The effects of enniatin B, B1, D, and enniatin mixture on the quantity of PDR5 protein as the translation product and on the stability of PDR5 protein were investigated by Western blotting of PDR5 protein. After addition of enniatin B, B1, D, or enniatin mixture, quantitative change of PDR5 protein was not observed, which confirmed that apparent inhibition of the function of PDR5 gene product is ascribable neither to inhibition of translation of PDR5 protein nor to reduced stability of the protein through acceleration of decomposition of PDR5 protein. These results could confirm that enniatins B inhibit, B1, D, and enniatin mixture directly act on PDR5 protein (PDR5 protein) to excretion of a drug such as cycloheximide and cerulenin to outside of cells.

### INDUSTRIAL APPLICABILITY

The ABC transporter inhibitor of the present invention inhibits transport or excretion of drugs, particularly anticancer drugs and antifungal agents, from inside to outside of cells, and therefore can prevent, suppress or inhibit acquisition of resistance against various drugs and agents. The ABC transporter inhibitor of the present invention is useful toprevent, suppress, or hinder acquisition of resistance against various agents, particularly anticancer drugs and antifungal agents.

## Claims

1. An ABC transporter inhibitor which comprises as an active ingredient a cyclic depsipeptide or its optical isomer or racemate of the formula (I): 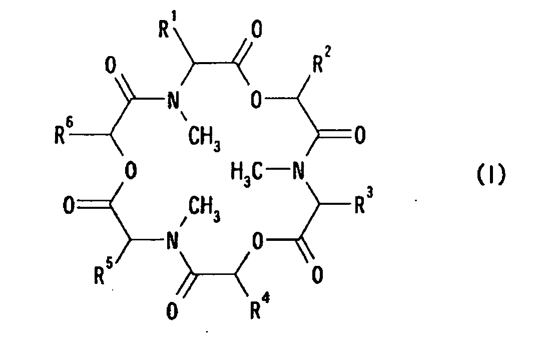 wherein R¹, R³ and R⁵ are each independently a group selected from linear or branched alkyl having up to 8 carbon atoms; hydroxyalkyl; alkanoyloxyalkyl; alkoxyalkyl; aryloxyalkyl; mercaptoalkyl; alkylthioalkyl; alkylsulfinylalkyl; alkylsulfonylalkyl; carboxyalkyl; alkoxycarbonylalkyl; arylalkoxycarbonylalkyl; carbamoylalkyl; aminoalkyl; alkylaminoalkyl; dialkylaminoalkyl; guanidinoalkyl; alkoxycarbonylaminoalkyl; 9-fluorenylmethoxycarbonyl(Fmoc)aminoalkyl; alkenyl; cycloalkyl; cycloalkylalkyl; and arylalkyl optionally substituted with halogen, hydroxy, alkyl, or alkoxy, and R², R⁴ and R⁶ are each independently a group selected from linear or branched alkyl having up to 8 carbon atoms; hydroxyalkyl; alkanoyloxyalkyl; alkoxyalkyl; aryloxyalkyl; alkylthioalkyl; alkylsulfinylalkyl; alkylsulfonylalkyl; carboxyalkyl; alkoxycarbonylalkyl; arylalkoxycarbonylalkyl; carbamoylalkyl; aminoalkyl; alkylaminoalkyl; dialkylaminoalkyl; alkoxycarbonylaminoalkyl; alkenyl; cycloalkyl; cycloalkylalkyl; and aryl or arylalkyl which are optionally substituted with halogen, hydroxy, alkyl, or alkoxy.

2. The ABC transporter inhibitor according to claim 1, wherein the cyclic depsipeptide is a compound of the formula (II): 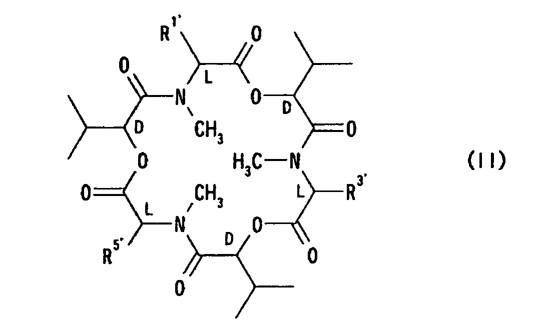 wherein R^{1'}, R^{3'} and R^{5'} are each independently linear or branched lower(C₁₋₄)alkyl.

3. The ABC transporter inhibitor according to claim 2, wherein the groups represented by R^{1'}, R^{3'} and R^{5'} are linear or branched propyl or butyl.

4. The ABC transporter inhibitor according to claim 3, wherein R^{1'} and R^{3'} are each isopyropyl, and R^{5'} is any one of the groups selected from isopropyl, sec-butyl, and isobutyl.

5. The ABC transporter inhibitor according to any one of claims 1 to 4, wherein the ABC transporter is MDR protein.

6. The ABC transporter inhibitor according to any one of claims 1 to 4, wherein the ABC transporter is CDR1 or CDR2 protein of *Candida* yeast.

7. The ABC transporter inhibitor according to any one of claims 1 to 4, wherein the ABC transporter is PDR5 protein of *Saccharomyces* yeast.

8. An inhibitor against the acquisition of drug resistance, which comprises as an active ingredient a cyclic depsipeptide or its optical isomer or racemate of the formula (I): 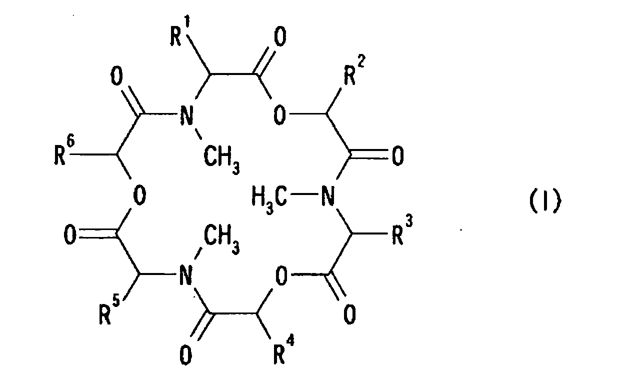 wherein R¹, R³ and R⁵ are each independently a group selected from linear or branched alkyl having up to 8 carbon atoms; hydroxyalkyl; alkanoyloxyalkyl; alkoxyalkyl; aryloxyalkyl; mercaptoalkyl; alkylthioalkyl; alkylsulfinylalkyl; alkylsulfonylalkyl; carboxyalkyl; alkoxycarbonylalkyl; arylalkoxycarbonylalkyl; carbamoylalkyl; aminoalkyl; alkylaminoalkyl; dialkylaminoalkyl; guanidinoalkyl; alkoxycarbonylaminoalkyl; 9-fluorenylmethoxycarbonyl(Fmoc)aminoalkyl; alkenyl; cycloalkyl; cycloalkylalkyl; and arylalkyl optionally substituted with halogen, hydroxy, alkyl, or alkoxy, and R², R⁴ and R⁶ are each independently a group selected from linear or branched alkyl having up to 8 carbon atoms; hydroxyalkyl; alkanoyloxyalkyl; alkoxyalkyl, aryloxyalkyl; alkylthioalkyl; alkylsulfinylalkyl; alkylsulfonylalkyl; carboxyalkyl; alkoxycarbonylalkyl; arylalkoxycarbonylalkyl; carbamoylalkyl; aminoalkyl; alkylaminoalkyl; dialkylaminoalkyl; alkoxycarbonylaminoalkyl; alkenyl; cycloalkyl; cycloalkylalkyl; and aryl or arylalkyl which are optionally substituted with halogen, hydroxy, alkyl, or alkoxy.
